# FASCICULE DE BREVET EUROPEEN

(11) **EP 1 371 692 B1**
(45) Date de publication et mention de la délivrance du brevet: **29.04.2009**
(21) Numéro de dépôt: 03291398.0
(22) Date de dépôt: 12.06.2003
(51) Int. Cl.: C09D 7/00

(54) **Microlatex inversé auto inversible, son procédé de préparation et ses utilisations**
Selbstinversierender inverser Microlatex, Verfahren zu seiner Herstellung und Verwendung
Auto inversible inverse Microlatex, process for its perparaion and its utilization

(30) Priorité: 13.06.2002 FR 0207257
(43) Date de publication de la demande: 17.12.2003
(73) Titulaire: SOCIETE D'EXPLOITATION DE PRODUITS POUR LES INDUSTRIES CHIMIQUES, S.E.P.P.I.C., 75321 Paris Cédex 07 (FR)
(72) Inventeur: Braun, Olivier, 81710 Naves (FR); Mallo, Paul, 78400 Chatou (FR); Tabacchi, Guy, 75007 Paris (FR)
(74) Mandataire: Conan, Philippe Claude

(56) Documents cités:
- EP-A- 1 010 708
- EP-A- 1 152 022
- EP-A- 1 152 023
- WO-A-00/32639
- FR-A- 2 774 688
- FR-A- 2 785 801
- US-B1- 6 375 959

## Description

La présente demande concerne des microlatex inverses auto-inversible eau dans huile épaississants, leur procédé de préparation et leur application en tant qu'épaississant et/ou émulsionnant pour des produits de soins de la peau et des cheveux ou pour la fabrication de préparations cosmétiques, dermopharmaceutiques ou pharmaceutiques, ou de formulations destinées au traitement des textiles.

Parmi les différents épaississants existants et utilisés pour épaissir des phases aqueuses, il y a les polymères sous forme de poudre tels que ceux décrits dans les demandes de brevet publiées sous les numéros EP 0341660, EP 0321650, EP 0341 662 ou encore les polymères décrits dans FR 2810545.

Parmi les différents épaississants existants et utilisés pour épaissir des phases aqueuses, il y a les émulsions inverses de polymères telles que celles décrites dans les demandes de brevet publiées sous les numéros EP 0161038, EP 0503853 ou FR 2790759.

Or la plupart de ces épaississants présentent l'inconvénient d'être encore très sensibles à la présence de sels.

A l'occasion de recherches sur la mise au point de nouveaux polymères plus résistants à la présence de sels, la demanderesse s'est plus particulièrement intéressée aux micro-émulsions inverses de polymères et a constaté que de façon inattendue, certaines d'entre elles possédaient un stabilité améliorée aux sels.

C'est pourquoi l'invention a pour objet une composition comprenant une phase huile, une phase aqueuse, au moins un agent tensioactif de type eau-dans-huile (E/H), au moins un agent tensioactif de type huile-dans-eau (H/E) et un polyélectrolyte branché ou réticulé, caractérisée en ce que,
(a) ladite composition est un microlatex inverse auto-inversible comprenant de 15 % à 40 % en poids, de préférence de 20 % à 30 % en poids dudit polyélectrolyte et entre 8 % et 20 % en poids, d'agents tensioactifs,
(b) ledit polyélectrolyté est, soit un homopolymère de l'acide 2-méthyl-2-[(1-oxo-2-propènyl) amino] 1-propanesulfonique partiellement ou totalement salifié, sous forme, soit d'un sel de métal alcalin, soit du sel d'ammonium, soit du sel de monéthanolamine ou soit d'un sel d'aminoacide., soit un copolymère à base d'acide 2-méthyl-2-[(1-oxo-2-propènyl) amino] 1-propanesulfonique partiellement ou totalement salifié, sous forme, soit d'un sel de métal alcalin, soit du sel d'ammonium, soit du sel de monéthanolamine ou soit d'un sel d'aminoacide, copolymérisé ou bien avec au moins un monomère possédant une fonction acide faible ou bien avec au moins un monomère neutre, soit un copolymère à base d'acide 2-méthyl-2-[(1-oxo-2-propènyl) amino] 1-propanesulfonique partiellement ou totalement salifié, sous forme, soit d'un sel de métal alcalin, soit du sel d'ammonium, soit du sel de monéthanolamine ou soit d'un sel d'aminoacide copolymérisé avec un monomère possédant au moins une fonction acide faible et avec au moins un monomère neutre,
   et
(c) le mélange dudit agent tensioactif de type eau-dans huile (E/H) et dudit agent tensioactif de type huile-dans-eau (H/E) a un nombre HLB total supérieur ou égal à 8,5 et inférieur ou égal à 11 et de préférence supérieur ou égal à 9,5 et inférieur ou égal à 10.

Par "agent tensioactif du type eau-dans-huile", on désigne des agents tensioactifs possédant une valeur de HLB suffisamment faible pour fournir des émulsions eau dans huile tels que les polymères tensioactifs du type copolymères séquencés polyéthylèneglycol poly(acide hydroxystéarique), commercialisés sous le nom HYPERMER®, tels que les esters de sorbitan, comme le mono-oléate de sorbitan commercialisé par la demanderesse sous le nom MONTANE 80™, l'isostéarate de sorbitan commercialisé par la demanderesse sous le nom MONTANE 70™, l'oléate de sorbitan éthoxylé avec 5 moles d'oxyde d'éthylène (5 OE) commercialisé par la demanderesse sous le nom MONTANE™ 81, l'alcool oléocétylique diéthoxylé (2 OE), commercialisé par la demanderesse sous le nom SIMULSOL™ OC 72 ou le sesquioléate de sorbitan commercialisé par la demanderesse sous le nom MONTANE™ 83.

Par "agent tensioactif du type huile-dans-eau", on désigne des agents tensioactifs possédant une valeur de HLB suffisamment élevée pour fournir des émulsions huile dans l'eau tels que les esters de sorbitan et éthoxylés comme l'oléate de sorbitan éthoxylé avec 20 moles d'oxyde d'éthylène (20 OE), commercialisé par la demanderesse sous le nom MONTANOX™ 80, l'huile de ricin éthoxylée à 40 moles d'oxyde d'éthylène (40 OE), commercialisée par la demanderesse sous le nom SIMULSOL™ OL 50, le laurate de sorbitan éthoxylé à 20 moles d'oxyde d'éthylène (20 OE), commercialisé par la demanderesse sous le nom MONTANO™ 20, le trioléate de sorbitan éthoxylé à 25 moles commercialisé par la demanderesse sous le nom MONTANOX™ 85, l'alcool laurique éthoxylé à 7 moles d'oxyde d'éthylène (7 OE), commercialisé par la demanderesse sous le nom SIMULSOL™ P 7, l'alcool oléocétylique décaéthoxylé (10 OE), commercialisé par la demanderesse sous le nom SIMULSOL™ OC 710 ou les hexaoléoates de sorbitan polyéthoxylés commercialisé sous les noms G-1086™ et G-1096™.

Au sens de la présente invention, le nombre HLB, est calculé par la formule HLB = 20 (1-Iₛ/Iₐ), dans laquelle Iₛ représente l'indice de saponification du tensioactif ou du mélange de tensioactifs et la, l'indice d'acide de l'acide gras de départ ou du mélange d'acides gras de départ, comme décrit par N. Schönfeld, dans le fascicule intitulé "Surface active ethylene oxide adducts" (page 228).

Par polymère branché, on désigne un polymère non linéaire qui possède des chaînes pendantes de manière à obtenir, lorsque ce polymère est mis en solution dans l'eau, un fort état d'enchevêtrement conduisant à des viscosités à bas gradient de vitesse très importantes.

Par polymère réticulé, on désigne un polymère non linéaire se présentant à l'état de réseau tridimensionnel insoluble dans l'eau, mais gonflable à l'eau et conduisant donc à l'obtention d'un gel chimique.

La fonction acide faible du monomère en comportant est notamment la fonction acide carboxylique, et de préférence, ledit monomère est choisi parmi l'acide acrylique, l'acide méthacrylique, l'acide itaconique l'acide maléique ou l'acide 3-méthyl 3-[(1-oxo 2-propènyl) amino] butanoïque, lesdits acides étant partiellement ou totalement salifié notamment sous forme, soit d'un sel de métal alcalin tel que par exemple le sel de sodium ou le sel de potassium, soit d'un sel d'ammonium, soit le sel d'un amino alcool tel que par exemple le sel de monoéthanolamine ou soit le sel d'un aminoacide tel que par exemple le sel de lysine.

Le monomère neutre est notamment choisi parmi l'acrylamide, l'acrylate de (2-hydroxy éthyle), l'acrylate de (2,3-dihydroxy propyle), le méthacrylate de (2-hydroxy éthyle), le méthacrylate de (2,3-dihydroxy propyle) ou un dérivé éthoxylé de poids moléculaire compris entre 400 et 1000, de chacun de ces esters.

Selon un premier aspect particulier de la présente invention, le polyélectrolyte compris dans le microlatex inverse auto-inversible tel que défini précédemment, est un homopolymère réticulé de l'acide 2-méthyl 2-[(1-oxo 2-propènyl) amino] 1-propanesulfonique partiellement ou totalement salifié sous forme de sel de sodium ou de sel d'ammonium.

Selon un deuxième aspect particulier de la présente invention, le polyélectrolyte compris dans le microlatex inverse auto-inversible tel que défini précédemment, est un copolymère réticulé de l'acide 2-méthyl 2-[(1-oxo 2-propènyl) amino] 1-propanesulfonique partiellement ou totalement salifié de sodium, de sel d'ammonium, de sel de monoéthanolamine ou de sel de lysine (a) et d'acrylate de (2-hydroxy éthyle) (b), dans un rapport molaire (a) / (b) compris entre 30 / 70 et 90 / 10 et tout particulièrement entre 50 / 50 et 90 / 10.

Selon un troisième aspect particulier de la présente invention, le polyélectrolyte compris dans le microlatex inverse auto-inversible, tel que défini précédemment, est un copolymère réticulé de sel de sodium, de sel d'ammonium, de sel de monoéthanolamine ou de sel de lysine de l'acide 2-méthyl 2-[(1-oxo 2-propènyl) amino] 1-propanesulfonique (a) et d'acide acrylique partiellement ou totalement salifié sous forme de sel de sodium, de sel d'ammonium, de sel de monoéthanolamine ou de sel de lysine (c), dans un rapport molaire (a) / (c), compris entre 30/70 et 90/10 et tout particulièrement entre 30 / 70 et 45 /55.

Selon un quatrième aspect particulier de la présente invention, le polyélectrolyte compris dans le microlatex inverse tel que défini précédemment, est un copolymère réticulé de sel de sodium ou de sel d'ammonium de l'acide 2-méthyl 2-[(1-oxo 2-propènyl) amino] 1-propanesulfonique (a) et d'acrylamide (d), dans un rapport molaire (a) / (d), compris entre 50 / 50 et 30 / 70.

Selon un cinquième aspect particulier de la présente invention, le polyélectroiyte compris dans le microlatex inverse auto-inversible, tel que défini précédemment, est un copolymère réticulé de sel de sodium, de sel d'ammonium, de sel de monoéthanolamine ou de sel de lysine de l'acide 2-méthyl 2-[(1-oxo 2-propènyl) amino] 1-propanesulfonique (a), d'acide acrylique partiellement ou totalement salifié sous forme de sel de sodium, de sel d'ammonium, de sel de monoéthanolamine ou de sel de lysine (c) et, soit d'acylamide (d), soit d'acrylate de (2-hydroxy éthyle) (e), dans des proportions molaires (a) (c) + (d) ou (a) / (c) + (e) comprises entre 10/ 90, 90 / 10 et (c) / (d) ou (c) / (e) comprises entre 10 / 90 et 90 / 10.

L'invention a plus particulièrement pour objet une composition telle que définie précédemment, caractérisée en ce que le polyélectrolyte est réticulé et/ou branché avec un composé diéthylénique ou polyéthylénique dans la proportion molaire exprimée par rapport aux monomères mis en oeuvre, de 0,005% à 1%, plus particulièrement de 0,01 % à 0,5 % et tout particulièrement ce 0,1 % à 0,25 %. L'agent de réticulation et/ou l'agent de ramification est choisi parmi l'acide diallyoxyacétique ou un de ses sels comme le diallyloxyacétate de sodium, le diméthacrylate d'éthylèneglycol, le diacrylate d'éthylèneglycol, le diallyl urée, le triméthylol propanetriacrylate, le méthyléne-bis(acrylamide), le triallylamine, le diallyl tartramide ou un mélange de ces composés.

La composition telle que définie précédemment, est caractérisée en ce que la phase huile représente de 25 % à 50%, et plus particutièrement de 30% à 40 %, de son poids total.

Cette phase huile est constituée, soit par une huile minérale commerciale contenant des hydrocarbures saturés, les paraffines, les isoparafines ou les cycloparaffines, présentant à température ambiante, une densité entre 0,7 et 0,9 et un point d'ébullition supérieur à 180°C, telle que par exemple l'EXXSOL™ D 100 S ou le MARCOL™52 commercialisés par EXXON CHEMICAL, l'isohexadécane ou l'isododécane, soit par une huile végétale, comme le squalane, soit une huile de synthèse comme le polyisobutène hydrogéné, soit par un mélange de plusieurs de ces huiles.

L'isohexadécane, qui est identifié dans Chemical Abstracts par le numéro RN = 93685-80-4, est un mélange d'isoparaffines en C₁₂, C₁₆ et C₂₀ contenant au moins 97 % d'isoparaffines en C₁₆, parmi lesquelles le constituant principal est le 2, 2, 4, 4, 6, 8, 8 - heptaméthyl nonane (RN = 4390-04-9). Il est commercialisé en France par la société BAYER. Le MARCOL™ 52 est une huile commerciale répondant à la définition des huiles de vaseline du Codex français. C'est une huile blanche minérale conforme aux réglementations FDA 21 CFR 172.878 et CFR 178.3620 (a) et elle est inscrite à la Pharmacopée des USA, US XXIII (1995) et à la Pharmacopée européenne (1993).

Les microlatex inverses auto-inversibles selon l'invention, contiennent entre 15 % et 50 % en poids d'eau.

Les microlatex inverses auto-inversibles selon l'invention, peuvent également contenir divers additifs tels que des agents complexant, des agents de transfert ou des agents limiteurs de chaîne.

Selon un autre aspect de la présente invention, celle-ci a pour objet un procédé de préparation d'une micro-émulsion inverse du polymère tel que défini précédemment, caractérisé en ce que :
a) - l'on ajoute sous agitation à une solution aqueuse contenant les monomères et les éventuels additifs, une phase huile et des agents tensioactifs, de façon à former une microémulsion inverse, puis
b) - l'on amorce la réaction de polymérisation, puis on laisse se dérouler ladite réaction pour former le microlatex inverse.

Selon une mise en oeuvre préférée du procédé tel que défini précédemment, la réaction de polymérisation est amorcée par un couple oxydoréducteur, tel que le couple hydroperoxyde de cumène - métabisulfite de sodium, à une température inférieure ou égale à 20°C, puis conduite soit de manière quasi-adiabatique jusqu'à une température supérieure ou égale à 40°C, plus particulièrement supérieure ou égale à 50°C, soit en contrôlant l'évolution de la température.

L'invention a aussi pour objet une composition cosmétique, dermopharmaceutique ou pharmaceutique, caractérisée en ce qu'elle comprend comme composé épaississant et/ou émulsionnant, au moins un microlatex inverse tel que défini précédemment.

La composition cosmétique, dermocosmétique, dermopharmaceutique ou pharmaceutique définie ci-dessus comprend généralement de 0,1 % à 10 % et plus particulièrement entre 0,5% et 5% en poids dudit microlatex inverse. Elle se présente notamment, sous la forme d'un lait, d'une lotion, d'un gel, d'une crème, d'un savon, d'un bain moussant, d'un baume, d'un shampooing ou d'un après shampooing.

De façon générale, ledit microlatex inverse, peut remplacer avantageusement les produits vendus sous le nom SEPIGEL™ 305 ou SEPIGEL™ 501 par la demanderesse, dans les compositions cosmétiques, dermopharmaceutiques ou pharmaceutiques, car il présente aussi une bonne compatibilité avec les autres excipients utilisés pour la préparation de formulations telles que les laits, les lotions, les crèmes, les savons, les bains, les baumes, les shampooings ou les après-shampooings. Il peut aussi être utilisé en combinaison lesdits SEPIGEL. Il est notamment compatible avec les concentrés décrits et revendiqués dans les publications internationales WO 92/06778, WO 95/04592, WO 95/13863 ou FR 2734 496 ou avec les agents tensioactifs décrits dans WO 93/08204. Il est particulièrement compatible avec le MONTANOV™ 68, le MONTANOV™ 82, le MONTANOV™ 202 le MONTANOV™ WO18, le MONTANOV™S ou le SEPIPERL™ N. Il peut également être utilisé dans des émulsions du type de celles décrites et revendiquées dans EP 0 629 396 et dans les dispersions aqueuses cosmétiquement ou physiologiquement acceptables avec un composé organopolysiloxane choisi, par exemple parmi ceux décrits dans WO 93/05762 ou dans WO 93/21316. Il peut également être utilisé pour former des gels aqueux à pH acide cosmétiquement ou physiologiquement acceptables, tels que ceux décrit dans WO 93/07856 ; il peut encore être utilisé en association avec des celluloses non-ioniques, pour former par exemple des gels de coiffage, tels que ceux décrits dans EP 0 684 024 ou encore en association avec des esters d'acides gras et de sucre, pour former des compositions pour le traitement du cheveu ou de la peau telles que celles décrites dans EP 0 603 019. ou encore dans les shampooings ou après shampooings tels que décrits et revendiqués dans WO 92/21316 ou enfin en association avec un homo polymère anionique tels que le CARBOPOL™ pour former des produits de traitement des cheveux comme ceux décrits dans DE 195 23596. Il est également compatible avec de nombreux principes actifs, tels que par exemple, les agents autobronzants comme le dihydroxyacétone (DHA) ou les agents anti-acné ; il peut donc être introduit dans des compositions auto-bronzantes comme celles revendiquées dans EP 0 715 845, EP 0604249, EP 0576188 ou dans WO 93/07902. II est également compatible avec les dérivés N-acylés d'aminoacides, ce qui permet son utilisation dans des compositions apaisantes notamment pour peaux sensibles, telles que celles décrites ou revendiquées dans WO 92/21318, WO 94/27561 ou WO 98/09611. Il est aussi compatible avec les acides glycoliques, avec l'acide lactique, avec l'acide salicylique les rétinoïdes, le phénoxy éthanol, les sucres, le glycéraldéhyde, les xanthanes, les acides de fruit, et les divers polyols utilisés dans la fabrication de formulations cosmétiques.

L'invention a donc aussi pour objet, l'utilisation d'un microlatex inverse tel que défini précédemment comme agent émulsionnant et / ou épaississant, dans la préparation d'une composition cosmétique, dermocosmétique, dermopharmaceutique ou pharmaceutique.

Selon un dernier aspect de la présente invention, celle-ci a pour objet, l'utilisation de la composition telle que définie précédemment comme épaississant et/ou émulsionnant dans les préparations destinées à toutes sortes d'industries qu'il s'agisse par exemple de l'industrie textile, de l'industrie du papier ou de la fabrication des peintures.

Selon un aspect particulier de ce dernier aspect de la présente invention, celle-ci a pour objet, l'utilisation d'un microlatex inverse auto-inversible comme agent épaississant des pâtes d'impression ou de la fabrication des peintures et plus particulièrement comme agent épaississant des pâtes d'impression à base de pigments ou de colorants. De telles pâtes sont notamment utilisées dans l'industrie textile. Les exemples qui suivent ont pour but d'illustrer la présente invention sans toutefois la limiter.

### Exemple 1: Préparation et propriétés du microlatex inverse (A) selon l'invention

### Préparation

On charge dans un bécher, sous agitation :
- 0,26 g de méthylène bis(acrylamide),
- 0,45 g d'une solution commerciale à 40 % en poids de diéthylènetriamine pentaacétate de sodium,
- 121,9 g d'une solution commerciale d'acrylamide à 50 % et
- 358,1 g d'une solution commerciale à 55 % en poids du sel de sodium de l'acide 2-méthyl-2-[(1-oxo-2-propènyl) amino] 1-propanesulfonique (ATBS Na 55 %).

Sur cette phase aqueuse, on ajoute successivement sous simple agitation :
- 393 g d'isohexadécane,
- 25, 2 g de Montane™ 80 VG,
- 116,2 g de Montanox™ 80,
- 0,41 g de d'AIBN (azobis(isobutyronitrile)
et on obtient une microémulsion inverse limpide.

La microémulsion obtenue est transférée dans un réacteur de polymérisation avec barbotage d'azote pendant une heure. On introduit alors 2,4 g d'une solution à 1 % en poids d'hydroperoxyde de cumène dans l'isohexadécane, puis on introduit alors une solution aqueuse de métabisulfite de sodium (0,2 g dans 100 ml d'eau) à raison de 0,5 ml/minute. L'introduction est réalisée pendant environ 10 minutes. On obtient en fin de polymérisation, un microlatex inverse auto-inversible stable et transparent.

### Evaluation des propriétés

Viscosité du microlatex inverse auto-inversible à 1 % de polymère dans l'eau (Brookfield RVT, axe 5 ; vitesse : 5 tours / mn) : η = 98 000 mPa s ;
Viscosité du microlatex inverse auto-inversible à 1 % de polymère dans l'eau + 0,1 % NaCI (Brookfield RVT, axe 5 ; vitesse : 5 tours / mn) : η = 29 400 mPa s ;
Ratio R : viscosité dans la solution salée / viscosité dans l'eau = 0,3.

A titre de comparaison, on a mesuré les viscosités a 1 % dans l'eau et à 1 % dans l'eau + 0,1 % NaCI, d'un latex inverse auto-inversible d'un copolymère réticulé préparé avec les mêmes monomères dans les mêmes proportions molaires (Acrylamide / ATBS : 50 / 50), le même agent de réticulation (méthylène bis(acrylamide), dans la même huile (isohexadécane). Les résultats sont consignés dans le tableau suivant :

| | microlatex inverse auto-inversible (invention) | latex inverse auto-inversible (état de la technique) |
|---|---|---|
| Viscosité à 1 % dans l'eau (RVT axe 6 vitesse 5) | 98 000 mPa s. | 74 800 mPa s |
| Viscosité à 1 % dans l'eau + 0,1 % Na Cl (RVT axe 5 vitesse 5) | 29 400 mPa s | 4 400 mPa s |
| Ratio R | 0.3 | 0.06 |

Ces résultats font apparaître que le microlatex inverse de l'invention est beaucoup plus stable au sel que le latex inverse du même copolymère dans la même huile à des concentrations similaires.

### Exemple 2: Préparation et propriétés du microlatex inverse (B) selon l'invention

### Préparation

On charge dans un bêcher, sous agitation :
- 0,40 g de méthylène bis(acrylamide),
- 0,45 g d'une solution commerciale à 40 % en poids de diéthylènetriamine pentaacétate de sodium,
- 0,55 g d'acide 2-méthyl-2-[(1-oxo-2-propènyl) amino] 1-propanesulfonique,
- 540 g d'une solution commerciale à 55 % en poids du sel de sodium de l'acide 2-méthyl-2-[(1-oxo-2-propènyl) amino] 1-propane sulfonique (ATBS Na 55 %).

Sur cette phase aqueuse, on ajoute successivement sous simple agitation :
- 360 g d'isohexadécane
- 17, 9 g de Montane™ 80 VG
- 82,9 g de Montanox™ 85
- 0,41 g d'AIBN.
et on obtient une microémulsion inverse limpide.

La micro-émulsion obtenue est transférée dans un réacteur de polymérisation avec barbotage d'azote pendant une heure. On introduit alors 2,4 g d'une solution à 1 % en poids d'hydroperoxyde de cumène dans l'isohexadécane, puis on introduit alors une solution aqueuse de métabisulfite de sodium (0,2 g dans 100 ml d'eau) à raison de 0,5 ml/minute. L'introduction est réalisée pendant environ 10 minutes. On obtient en fin de polymérisation, un microlatex inverse auto-inversible stable et transparent.

### Evaluation des propriétés

Viscosité du microlatex inverse auto-inversible à 1 % de polymère dans l'eau (Brookfield RVT, axe 5 ; vitesse : 5 tours / mn) : η = 52 000 mPa.s ;
Viscosité du microlatex inverse auto-inversible à 1 % de polymère dans l'eau + 0,1 % NaCI (Brookfield RVT, axe 5 ; vitesse : 5 tours / mn) : η = 15 000 mPa.s ;
Ratio : viscosité dans la solution salée / viscosité dans l'eau = 0,3.

A titre de comparaison, on a mesuré les viscosités à 1 % dans l'eau et à 1% dans l'eau + 0,1 % NaCI, d'un latex inverse auto-inversible d'un hompolymère réticulé préparé avec le même monomère, le même agent de réticulation (méthylène bis(acrylamide), dans la même huile (isohexadécane). Les résultats sont consignés dans le tableau suivant :

| | microlatex inverse auto-inversible (invention) | latex inverse auto-inversible (état de la technique) |
|---|---|---|
| Viscosité à 1 % dans l'eau (RVT axe 6 vitesse 5) | 52 000 mPa s. | 100 000 mPa s |
| Viscosité à 1 % dans L'eau + 0,1 % NaCl (RVT axe 5 vitesse 5) | 15 000 mPa s | 6 200 mPa s |
| Ratio R | 0.3 | 0.06 |

Ces résultats font apparaître que le microlatex inverse de l'invention est beaucoup plus stable au sel que le latex inverse du même homopolymère dans la même huile à des concentrations similaires.

### Exemple 3: Préparation et propriétés du microlatex inverse (C) selon l'invention

### Préparation

On charge dans un bêcher, sous agitation :
- 0,26 g de méthylène bis(acrylamide),
- 0,45 g d'une solution commerciale à 40 % en poids de diéthylènetriamine pentaacétate de sodium,
- 120,2 g d'une solution commerciale d'acrylamide à 50 %
- 352,8 g d'une solution commerciale à 55 % en poids du sel de sodium de l'acide 2-méthyl-2-[(1-oxo-2-propènyl) amino] 1-propane sulfonique (ATBS Na 55 %) et,
- 0,4 g d'acide 2-méthyl-2-[(1-oxo-2-propényl) amino] 1-propanesulfonique,

Sur cette phase aqueuse, on ajoute successivement sous simple agitation :
- 387,1 g de polyisobutène hydrogénén (PIB),
- 39,7 g de Montane™ 80 VG,
- 100,3 g de Montanox™ 85
- 0,4 g d'AIBN,
et on obtient une microémulsion inverse limpide.

La micro-émulsion obtenue est transférée dans un réacteur de polymérisation avec barbotage d'azote pendant une heure. On introduit alors 2,4 g d'une solution à 1 % en poids d'hydroperoxyde de cumène dans le PIB, puis on introduit alors une solution aqueuse de métabisulfite de sodium (0,2 g dans 100 ml d'eau) à raison de 0,5 ml/minute. L'introduction est réalisée pendant environ 10 minutes.

On obtient en fin de polymérisation, un microlatex inverse auto-inversible stable et transparent.

### Evaluation des propriétés

Viscosité du microlatex inverse auto-inversible à 1 % de polymère dans l'eau (Brookfield RVT, axe 6 ; vitesse : 5 tours / mn) : η = 72 000 mPas s ;
Viscosité du microlatex inverse auto-inversible à 1 % de polymère dans l'eau + 0,1 % NaCI (Brookfield RVT, axe 6 ; vitesse : 5 tours / mn) : η = 21 000 mPa s ;
Ratio : viscosité dans la solution salée / viscosité dans l'eau = 0,3

### C) Exemples de formulations préparées avec les compositions selon l'invention

### Exemple 4 : Crème de soin

| | |
|---|---|
| Cyclométhicone | 10 % |
| Microlatex inverse A | 0,8 % |
| MONTANOV™ 68 | 2% |
| alcool stéarylique | 1% |
| alcool stéarique | 0,5% |
| conservateur | 0,65 % |
| Lysine | 0,025 % |
| EDTA (sel disodique) | 0,05 % |
| Gomme de xanthane | 0,2 % |
| Glycérine | 3% |
| Eau | q.s.p. 100% |

### Exemple 5 : Baume après-rasage

| FORMULE | | |
|---|---|---|
| A | Microlatex inverse A | 1,5 % |
| | Eau | q.s.p. 100% |
| B | MICROPEARL™ M 100 | 5,0 % |
| | SEPICIDE™ Cl | 0,50 % |
| | Parfum | 0,20% |
| | éthanol 95° | 10,0 % |

### MODE OPERATOIRE

Ajouter B dans A.

### Exemple 6 : Emulsion satinée pour le corps

| FORMULE | | |
|---|---|---|
| A | SIMULSOL™ 165 | 5,0% |
| | LANOL™ 1688 | 8,50% |
| | beurre de Karité | 2% |
| | huile de paraffine | 6,5% |
| | LANOL™ 14M | 3% |
| | LANOL™ S | 0,6 % |
| B | eau | 66,2% |
| C | MICROPEARL™ M 100 | 5 % |
| D | Microlatex inverse C | 3 % |
| E | SEPICIDE™ Cl | 0,3 % |
| | SEPICIDE™ HB | 0,5 % |
| | MONTEINE™ CA | 1% |
| | Parfum | 0,20% |
| | acétate de vitamine E | 0,20 % |
| | Sodium pyrolidinonecarboxylate | 1 % |

### MODE OPERATOIRE

Ajouter C dans B, émulsionner B dans A à 70°C, puis ajouter D à 60°C puis E à 30°C.

### Exemple 7 : Crème H/E

| FORMULE | | |
|---|---|---|
| A | SIMULSOL™165 | 5,0% |
| | LANOL™ 1688 | 20,0% |
| | LANOL™ P | 1,0% |
| B | eau | q.s.p. 100% |
| C | Microlatex inverse C | 2,50 % |
| D | SEPICIDE™ Cl | 0,20 % |
| | SEPICIDE™ HB | 0,30 % |

### MODE OPERATOIRE

Introduire B dans A vers 75°C ; ajouter C vers 60°C, puis D vers 45°C.

### Exemple 8 : gel solaire non gras

| FORMULE | | |
|---|---|---|
| A | Microlatex inverse B | 3,00 % |
| | Eau | 30% |
| B | SEPICIDETM Cl | 0,20 % |
| | SEPICIDE™ HB | 0,30 % |
| | Parfum | 0,10% |
| C | colorant | q.s.p. |
| | eau | 30% |
| D | MICROPEARL™ M 100 | 3,00% |
| | Eau | q.s.p. 100% |
| E | huile de silicone | 2,0 % |
| | PARSOL™ MCX | 5,00 % |

### MODE OPERATOIRE

Introduire B dans A; ajouter C, puis D, puis E.

### Exemple 9 : Lait solaire

| FORMULE | | |
|---|---|---|
| A | MONTANOV™ S | 3,0% |
| | huile de sésame | 5,0% |
| | PARSOLTM MCX | 5,0% |
| | Carraghénane λ | 0,10 % |
| B | eau | q.s.p. 100% |
| C | Microlatex inverse A | 0,80 % |
| D | Parfum | q. s. |
| | Conservateur | q. s. |

### MODE OPERATOIRE

Emulsionner B dans A à 75°C puis ajouter C vers 60°C, puis D vers 30°C et ajuster le pH si nécessaire

### Exemple 10 : Gel de massage

| FORMULE | | |
|---|---|---|
| A | Microlatex inverse B | 3,5 % |
| | Eau | 20,0% |
| B | colorant | 2 gouttes/100g |
| | Eau | q.s. |
| C | alcool | 10% |
| | Menthol | 0,10% |
| D | huile de silicone | 5,0% |

### MODE OPERATOIRE

Ajouter B dans A, puis ajouter au mélange, C puis D

### Exemple 11 : Fond de teint hydratant et matifiant

| FORMULE | | |
|---|---|---|
| A | eau | 20,0% |
| | Butylène glycol | 4,0 % |
| | PEG-400 | 4,0 % |
| | PECOSIL™ PS100 | 1,0% |
| | NaOH | q.s. pH = 9 |
| | Dioxyde de titane | 7,0% |
| | Talc | 2,0% |
| | Oxyde de fer jaune | 0,8 % |
| | Oxyde de fer rouge | 0,3 % |
| | Oxyde de fer noir | 0,05 % |
| B | LANOL™ 99 | 8% |
| | Caprylic capric triglycéride | 8 % |
| | MONTANOV™ 202 | 5,00 % |
| C | eau | q.s.p. 100% |
| | MICROPEARL™ M305 | 2,0 % |
| | EDTA tétrasodé | 0,05 % |
| D | Cyclométhicone | 4,0 % |
| | Gomme de Xanthane | 0,2 % |
| | Microlatex inverse A | 0,8 % |
| E | SEPICIDE™ HB | 0,5 % |
| | SEPICIDE Cl | 0,3 % |
| | Parfum | 0,2% |

### MODE OPERATOIRE

préparer à 80°C, les mélanges B + D et A + C, puis mélanger et émulsionner l'ensemble.

### Exemple 12 : Gel coup d'éclat

| FORMULE | | |
|---|---|---|
| A | Microlatex inverse C : | 4 % |
| | Eau : | 30% |
| B | ELASTINE HPM : | 5,0 % |
| C | MICROPEARL™ M 100 : | 3 % |
| | Eau : | 5% |
| D | SEPICIDE™ Cl : | 0,2 % |
| | SEPICIDE™ HB : | 0,3 % |
| | Parfum : | 0,06% |
| Sodium pyrolidinonecarboxylate 50% : | | 1 % |
| Eau : | | q.s.p. 100% |

### MODE OPERATOIRE

Préparer A ; additionner B, puis C, puis D.

### Exemple 13 : Lait corporel

| FORMULE | |
|---|---|
| MONTANOV™ S | 3,5% |
| LANOL™ 37T | 8,0% |
| SOLAGUM™ L | 0,05 % |
| Eau | q.s.p.100% |
| Benzophénone | 2,0 % |
| diméthicone 350cPs | 0,05 % |
| Microlatex inverse B | 0,8 % |
| conservateur | 0,2% |
| parfum | 0,4% |

### Exemple 14 : Emulsion démaquillante à l'huile d'amandes douces

| FORMULE | |
|---|---|
| MONTANOV™ 68 | 5% |
| huile d'amandes douces | 5 % |
| eau | q.s.p. 100% |
| Microlatex inverse A | 0,3 % |
| glycérine | 5% |
| conservateur | 0,2% |
| parfum | 0,3% |

### Exemple 15 : Crème hydratante pour peaux grasses

| FORMULE | |
|---|---|
| MONTANOV™ 68 | 5% |
| Cétylstéaryloctanoate | 8 % |
| octyl palmitate | 2% |
| eau | q.s.p. 100% |
| Microlatex inverse C | 0,6 % |
| MICROPEARL™ M100 | 3,0 % |
| Mucopolysaccharides | 5 % |
| SEPICIDE™ HB | 0,8 % |
| Parfum | 0,3 % |

### Exemple 16 : Baume après-rasage apaisant sans alcool

| FORMULE | | |
|---|---|---|
| A | LIPACIDE™ PVB | 1,0% |
| | LANOL™ 99 | 2,0 % |
| | Huile d'amandes douces | 0,5 % |
| B | Microlatex inverse A | 3,5 % |
| C | eau | q.s.p. 100% |
| D | parfum | 0,4 % |
| | SEPICIDE™ HB | 0,4 % |
| | SEPICIDE™ Cl | 0,2 % |

### Exemple 17 : Crème aux AHA pour peaux sensibles

| FORMULE | |
|---|---|
| mélange de lauryl aminoacides | 0,1% à 5 % |
| aspartate de magnésium et de potassium | 0,002% à 0,5 % |
| LANOL™ 99 | 2% |
| MONTANOV™ 68 | 5,0 % |
| Eau | q.s.p. 100 % |
| Microlatex inverse B | 1,50 % |
| acide gluconique | 1,50 % |
| tri éthylamine | 0,9 % |
| SEPICIDE™ HB | 0,3 % |
| SEPICIDE™ Cl | 0,2 % |
| Parfum | 0,4% |

### Exemple 18 : Soin apaisant après soleil

| FORMULE | |
|---|---|
| mélange de lauryl aminoacides | 0,1 % à 5 % |
| aspartate de magnésium et de potassium | 0,002 % à 0,5 % |
| LANOL™ 99 | 10,0% |
| Eau | q.s.p. 100% |
| Microlatex inverse A | 2,50 % |
| SEPICIDE™ HB | 0,3 % |
| SEPlCIDETM Cl | 0,2 % |
| Parfum | 0,4 % |
| Colorant | 0,03% |

### Exemple 19 : Lait démaquillant

| FORMULE | |
|---|---|
| SEPIPERL™ N | 3% |
| PRIMOL™ 352 | 8,0 % |
| huile d'amandes douces | 2 % |
| eau | q.s.p. 100% |
| Microlatex inverse B | 0,8 % |
| conservateur | 0,2 % |

### Exemple 20 : Emulsion fluide à pH alcalin

| | |
|---|---|
| MARCOL™ 82 | 5,0% |
| NaOH | 10,0% |
| Eau | q.s.p. 100% |
| Microlatex inverse A | 1,5 % |

### Exemple 21 : Fond de teint fluide

| FORMULE | |
|---|---|
| SIMULSOL™ 165 | 5,0% |
| LANOL™ 84D | 8,0 % |
| LANOL™ 99 | 5,0 % |
| Eau | q.s.p. 100% |
| pigments et charges minérales | 10,0 % |
| Microlatex inverse A | 1,2 % |
| conservateur | 0,2% |
| parfum | 0,4% |

### Exemple 22 : Lait solaire

| FORMULE | |
|---|---|
| SEPIPERL™ N | 3,5 % |
| LANOL™ 37T | 10,0% |
| PARSOL™ MCX | 5,0 % |
| EUSOLEX™ 4360 | 2,0 % |
| Eau | q.s.p. 100% |
| Microlatex inverse A | 1,8% |
| conservateur | 0,2% |
| parfum | 0,4% |

### Exemple 23 : Gel contour des yeux

| FORMULE | |
|---|---|
| Microlatex inverse A | 2,0 % |
| Parfum | 0,06% |
| Sodium pyrrolidinonecarboxylate | 0,2 % |
| DOW CORNING™ 245 Fluid | 2,0 % |
| Eau | q.s.p. 100% |

### Exemple 24 : Composition de soin non rincée

| FORMULE | |
|---|---|
| Microlatex inverse C | 1,5 % |
| Parfum | q.s |
| Conservateur | q.s |
| DOW CORNING™ X2 8360 | 5,0 % |
| DOW CORNING™ Q2 1401 | 15,0 % |
| Eau | q.s.p. 100% |

### Exemple 25 : Gel amincissant

| | |
|---|---|
| Microlatex inverse C | 5 % |
| Ethanol | 30 % |
| Menthol | 0,1 % |
| Caféine | 2,5 % |
| extrait de ruscus | 2% |
| extrait de lierre | 2% |
| SEPICIDE™ HP | 1% |
| Eau | q.s.p. 100% |

### Exemple 26 : Gel crème teinté ultra naturel

| FORMULE | | |
|---|---|---|
| A | eau | 10,0% |
| | Butylène glycol | 4,0% |
| | PEG-400 | 4,0% |
| | PECOSIL^{TM} PS 100 | 1,5% |
| | NaOH | q.s. pH = 7 |
| | Dioxyde de titane | 2,0% |
| | Oxyde de fer jaune | 0,8% |
| | Oxyde de fer rouge | 0,3% |
| | Oxyde de fer noir | 0,05% |
| B | LANOL™ 99 | 4,0% |
| | Caprylic capric triglycéride | 4,0% |
| | SEPIFEEL™ ONE | 1,0% |
| | Microlatex inverse B | 3,0% |
| C | eau | q.s.p. 100% |
| | MICROPEARL™ M305 | 2,0% |
| | EDTA tétrasodé | 0,05% |
| | Cyclométhicone | 4,0% |
| D | SEPICIDE™ HB | 0,5% |
| | SEPICIDE Cl | 0,3% |
| | Parfum | 0,2% |

### MODE OPERATOIRE

préparer le mélange B + C puis ajouter A puis D.

### Exemple 27 : Soin pour les peaux grasses

| FORMULE | | |
|---|---|---|
| A | MICROPEARL™ M310 | 1,0% |
| | Microlatex inverse A | 5,0 % |
| | Isononanoate d'octyle | 4.0 % |
| B | eau | q.s.p. 100% |
| C | SEPICONTROL™ A5 | 4,0 % |
| | Parfum | 0,1 % |
| | SEPICIDE™ HB | 0,3 % |
| | SEPICIDE™ Cl | 0,2 % |
| D | CAPIGEL™ 98 | 0,5 % |
| | Eau | 10% |

### Exemple 28 : Crème aux AHA

| FORMULE | | |
|---|---|---|
| A | MONTANOV™ 68 | 5,0 % |
| | LIPACIDE™ PVB | 1,05% |
| | LANOL™ 99 | 10,0% |
| B | eau | q.s.p. 100% |
| | Acide gluconique | 1,5 % |
| | TEA (triéthanolamine) | 0,9 % |
| C | Microlatex inverse B | 1,5 % |
| D | parfum | 0,4 % |
| | SEPICIDE™ HB | 0,2 % |
| | SEPICIDE™ Cl | 0,4% |

### Exemple 29 : Autobronzant non gras pour visage et corps

| FORMULE | | |
|---|---|---|
| A | LANOL™ 2681 | 3,0 % |
| | Microlatex inverse A | 2,5 % |
| B | eau | q.s.p. 100% |
| | Dihydroxyacétone | 3,0 % |
| C | parfum | 0,2% |
| | SEPICIDE™ HB | 0,8 % |
| | NaOH (hydroxyde de sodium) : | q.s pH = 5 |

### Exemple 30 : Lait solaire au monoï de Tahiti

| FORMULE | | |
|---|---|---|
| A | Monoï de Tahiti | 10% |
| | LIPACIDE™ PVB | 0,5 % |
| | Microlatex inverse B | 2,2 % |
| B | eau | q.s.p. 100% |
| C | parfum | 0,1 % |
| | SEPICIDE™ HB | 0,3 % |
| | SEPICIDE™ Cl | 0,1 % |
| | PARSOL™ MCX | 4,0 % |

### Exemple 31 : Soin solaire pour le visage

| FORMULE | | |
|---|---|---|
| A | Cyclométhicone et diméthiconol | 4,0 % |
| | Microlatex inverse B | 3,5 % |
| B | eau | q.s.p. 100% |
| C | parfum | 0,1 % |
| | SEPICIDE™ HB | 0,3 % |
| | SEPICIDE™ Cl | 0,21 % |
| | PARSOL™ MCX | 5,0% |
| | Micatitane | 2,0 % |
| | Acide lactique | q.s.p. pH = 6,5 |

### Exemple 32 : Emulsion bronzante sans soleil

| FORMULE | | |
|---|---|---|
| A | LANOL™ 99 | 15% |
| | MONTANOV™ 68 | 5,0 % |
| | PARSOL™ MCX | 3,0% |
| B | eau | q.s.p. 100% |
| | Dihydroxyacétone | 5,0 % |
| | Phosphate monosodique | 0,2 % |
| C | Microlatex inverse A | 0,5 % |
| D | parfum | 0,3% |
| | SEPICIDE™ HB | 0,8 % |
| | NaOH | q.s. pH=5. |

Les caractéristiques des produits utilisés dans les exemples précédents, sont les suivantes :
Le MONTANOV™ 68 (cétéaryl glucoside, alcool cétéarylique), est une composition auto-émulsionnable telle que celles décrites dans WO 92/06778, commercialisée par la société SEPPIC.
Le MONTANOV™ 202 (arachidyl glucoside, alcool arachydilique + alcool béhènylique), est une composition auto-émulsionnable telle que celles décrites dans WO 98/17610, commercialisée par la société SEPPIC.
Le MICROPEARL™ M 305 est une poudre hydrodispersible soyeuse à base de copolymer méthylméthacrylate réticulé.
Le MICROPEARL™ M 100 est une poudre ultra fine au toucher très doux et à action matifiante commercialisée par la société MATSUMO
Le SEPICIDE™ Cl, imidazoline urée, est un agent conservateur commercialisé par la société SEPPIC.
PEMULEN™ TR est un polymère acrylique commercialisé par GOODRICH.
Le SIMULSOL™ 165 est du stéarate de glycérol auto-émulsionnable commercialisée par la société SEPPIC.
Le LANOL™ 1688 est un ester émollient à effet non gras commercialisé par la société SEPPIC.
Le LANOL™ 14M et le LANOL ™ S sont des facteurs de consistance commercialisés par la société SEPPIC.
Le SEPICIDE™ HB , qui est un mélange de phénoxyéthanol, de méthylparaben, d'éthylparaben, de propylparaben et de butylparaben, est un agent conservateur commercialisé par la société SEPPIC.
Le MONTEINE™ CA est un agent hydratant commercialisé par la société SEPPIC.
Le SCHERCEMOL™ OP est un ester émollient à effet non gras.
   Le LANOL™ P est un additif à effet stabilisant commercialisé par la société SEPPIC.
Le SEPIPERL™ N est un agent nacrant, commercialisé par la société SEPPIC, à base d'un mélange d'alkyl polyglucosides tels que ceux décrits dans WO 95/13863.
Le MONTANOV™ S est un agent nacrant, commercialisé par la société SEPPIC, à base d'un mélange d'alkyl polyglucosides tels que ceux décrits dans WO 95/13863.
Le PECOSIL™ PS100 est du diméthicone copolyol phosphate commercialisé par la société PHOENIX.
Le LANOL™ 99 est de l'isononyl isononanoate commercialisé par la société SEPPIC.
Le LANOL™ 37T est du triheptanoate de glycérol, commercialisé par la société SEPPIC.
Le SEPIFEEL™ ONE est un mélange de palmitoylproline, de palmitoyl glutamate de magnésium et de palmitoyl sarcosinate de magnésium, tel que ceux décrits dans FR 2787323.
Le SOLAGUM™ L est un carraghénane commercialisé par la société SEPPIC.
Le MARCOL™ 82 est une huile de paraffine commercialisée par la société ESSO.
Le LANOL™ 84D est du malate de dioctyle commercialisé par la société SEPPIC.
Le PARSOL™ MCX est du paraméthoxycinnamate d'(éthyl hexyle) commercialisé par la société GIVAUDAN.
L'EUSOLEX™ 4360 est de la benzophénone-3 commercialisé par la société MERCK.
Le DOW CORNING™ 245 Fluid est de la cyclométhicone, commercialisée par la société DOW CORNING.
Le LIPACIDE™ PVB,est un hydrolysat de protéines de blé palmitoylé, est commercialisée par la société SEPPIC.
Le SEPICONTROL™ A5 est un mélange capryloyl glycine, sarcosine, extrait de cinnamon zylanicum, commercialisé par la société SEPPIC, tel que ceux décrits dans la demande internationale de brevet PCT/FR98/01313 déposée le 23 juin 1998.
Le CAPIGEL™ 98 est un copolymère d'acrylates commercialisé par la société SEPPIC.
Le LANOL™ 2681 est un mélange caprylate, caprate de coprah, commercialisé par la société SEPPIC.

## Revendications

1. Composition comprenant une phase huile, une phase aqueuse, au moins un agent tensioactif de type eau-dans-huile (E/H), au moins un agent tensioactif de type huile-dans-eau (H/E) et un polyélectrolyte branché ou réticulé, **caractérisée en ce que** :
(a) ladite composition est un microlatex inverse auto-inversible comprenant de 15 % à 40 % en poids, de préférence de 20 % à 30 % en poids dudit polyélectrolyte et entre 8 % et 20 % en poids, d'agents tensioactifs,
(b) ledit polyélectrolyte est, soit un homopolymère de l'acide 2-méthyl-2-[(1-oxo-2-propènyl) amino] 1-propanesulfonique partiellement ou totalement salifié, sous forme, soit d'un sel de métal alcalin, soit du sel d'ammonium, soit du sel de monéthanolamine ou soit d'un sel d'aminoacide., soit un copolymère à base d'acide 2-méthyl-2-[(1-oxo-2-propènyl) amino] 1-propanesulfonique partiellement ou totalement salifié, sous forme, soit d'un sel de métal alcalin, soit du sel d'ammonium, soit du sel de monéthanolamine ou soit d'un sel d'aminoacide, copolymérisé ou bien avec au moins un monomère possédant une fonction acide faible ou bien avec au moins un monomère neutre, soit un copolymère à base d'acide 2-méthyl-2-[(1-oxo-2-propènyl) amino] 1-propanesulfonique partiellement ou totalement salifié, sous forme, soit d'un sel de métal alcalin, soit du sel d'ammonium, soit du sel de monéthanolamine ou soit d'un sel d'aminoacide copolymérisé avec un monomère possédant au moins une fonction acide faible et avec au moins un monomère neutre, et
(c) le mélange dudit agent tensioactif de type eau-dans huile (E/H) et dudit agent tensioactif de type huile-dans-eau (H/E) a un nombre HLB total supérieur ou égal à 8,5 et inférieur ou égal à 11 et de préférence supérieur ou égal à 9,5 et inférieur ou égal à 10.

2. Composition tel que définie à la revendication 1 dans laquelle le monomère comportant une fonction acide faible, est choisi parmi l'acide acrylique, l'acide méthacrylique, l'acide itaconique, l'acide maléique ou l'acide 3-méthyl 3-[(1-oxo 2-propènyl) amino] butanoïque, lesdits acides étant partiellement ou totalement salifiés sous forme, soit d'un sel de métal alcalin, soit du sel d'ammonium, soit du sel de monéthanolamine ou soit d'un sel d'aminoacide.

3. Composition telle que définie à la 1, dans laquelle le monomère neutre est choisi parmi l'acrylamide, l'acrylate de (2-hydroxy éthyle), l'acrylate de (2,3-dihydroxy propyle), le méthacrylate de (2-hydroxy éthyle), le méthacrylate de (2,3-dihydroxy propyle), ou un dérivé éthoxylé de poids moléculaire compris entre 400 et 1000, de chacun de ces esters.

4. Composition telle que définie à la revendication 1, dans laquelle le polyélectrolyte est un homopolymère réticulé de l'acide 2-méthyl 2-[(1-oxo 2-propènyl) amino] 1-propanesulfonique partiellement ou totalement salifié sous forme de sel de sodium ou de sel d'ammonium.

5. Composition telle que définie à l'une des revendications 1 ou 3, dans laquelle le polyélectrolyte est un copolymère réticulé de l'acide 2-méthyl 2-[(1-oxo 2-propènyl) amino] 1-propanesulfonique partiellement ou totalement salifié sous forme du sel de sodium, de sel d'ammonium, de sel de monoéthanolamine ou de sel de lysine (a) et d'acrylate de (2-hydroxy éthyle) (b), dans un rapport molaire (a) / (b) compris entre 30 / 70 et 90 / 10 et tout particulièrement entre 50 / 50 et 90 / 10.

6. Composition telle que définie à l'une des revendications 1 ou 2, dans laquelle le polyélectrolyte est un copolymère réticulé de sel de sodium, de sel d'ammonium, de sel de monoéthanolamine ou de sel de lysine de l'acide 2-méthyl 2-[(1-oxo 2-propènyl) amino] 1-propanesulfonique (a) et d'acide acrylique partiellement ou totalement salifié sous forme de sel de sodium, de sel d'ammonium, de sel de monoéthanolamine ou de sel de lysine (c), dans un rapport molaire (a) / (c), compris entre 30 / 70 et 90/10 et tout particulièrement entre 30 / 70 et 45 / 55.

7. Composition telle que définie à l'une des revendications 1 ou 3, dans laquelle le polyélectrolyte est un copolymère réticulé de sel de sodium ou de sel d'ammonium de l'acide 2-méthyl-2-[(1-oxo-2-propènyl) amino] 1-propanesulfonique (a) et d'acrylamide (d), dans un rapport molaire (a) / (d), compris entre 50 / 50 et 30 / 70.

8. Composition telle que définie à la revendication 1, dans laquelle le polyélectrolyte est un copolymère réticulé de sel de sodium, de sel d'ammonium, de sel de monoéthanolamine ou de sel de lysine de l'acide 2-méthyl 2-[(1-oxo 2-propènyl) amino] 1-propanesulfonique (a), d'acide acrylique partiellement ou totalement salifié sous forme de sel de sodium, de sel d'ammonium, de sel de monoéthanolamine ou de sel de lysine (c) et, soit d'acylamide (d), soit d'acrylate de (2-hydroxy éthyle) (e), dans des proportions molaires (a) / (c) + (d) ou (a) / (c) + (e) comprises entre 10 / 90, 90 /10 et (c) / (d) ou (c) / (e) comprises entre 10/90 et 90 / 10.

9. Composition telle que définie à l'une des revendications 1 à 8, dans laquelle, **caractérisée en ce que** le polyélectrolyte est réticulé et/ou branché avec un composé diéthylénique ou polyéthylénique dans la proportion molaire exprimée par rapport aux monomères mis en oeuvre, de 0,005% à 1%, plus particulièrement de 0,01 % à 0,5 % et tout particulièrement de 0,1 % à 0,25 %.

10. Composition telle que définie à la revendication 8, dans laquelle l'agent de réticulation et/ou l'agent de ramification est choisi parmi l'acide diallyoxyacétique ou un de ses sels comme le diallyloxyacétate de sodium, le diméthacrylate d'éthylèneglycol, le diacrylate d'éthylèneglycol, le diallyl urée, le triméthylol propanetriacrylate, le méthylène-bis(acrylamide), le triallylamine, le diallyltartramide ou un mélange de ces composés.

11. Composition telle que définie à l'une des revendications 1 à 10, dans laquelle la phase huile représente de 25 % à 50%, et plus particulièrement de 30% à 40 %, de son poids total.

12. Procédé de préparation d'une composition telle que définie à l'une des revendications 1 à 11, **caractérisé en ce que** :
a) - l'on ajoute sous agitation à une solution aqueuse contenant les monomères et les éventuels additifs, une phase huile et des agents tensioactifs, de façon à former une microémulsion inverse, puis
b) -l'on amorce la réaction de polymérisation, puis on laisse se dérouler ladite réaction pour former le microlatex inverse.

13. Composition cosmétique, dermopharmaceutique ou pharmaceutique, **caractérisée en ce qu'**elle comprend au moins une composition telle que définie à l'une des revendications 1 à 11.

14. Utilisation d'une composition telle que définie à l'une des revendications 1 à 11 comme épaississant et/ou émulsionnant dans les préparations destinées à toutes sortes d'industries, qu'il s'agisse par exemple de l'industrie textile, de l'industrie du papier ou de la fabrication des peintures et plus particulièrement comme agent épaississant des pâtes d'impression à base de pigments ou de colorants.

## Claims

1. Composition comprising an oil phase, an aqueous phase, at least one surfactant of water-in-oil (W/O) type, at least one surfactant of oil-in-water (O/W) type and a branched or crosslinked polyelectrolyte, **characterized in that**:
(a) the said composition is a self-reversible reverse microlatex comprising from 15% to 40% by weight and preferably from 20% to 30% by weight of the said polyelectrolyte, and between 8% and 20% by weight of surfactants,
(b) the said polyelectrolyte is either a homopolymer 2-methyl-2-[(1-oxo-2-propenyl)amino]-1-propanesulphonic acid partially or totally salified in the form either of an alkali metal salt, or of the ammonium salt, or of the monoethanolamine salt or of an amino acid salt, or a copolymer based on 2-methyl-2-[(1-oxo-2-propenyl)amino]-1-propanesulphonic acid partially or totally salified in the form either of an alkali metal salt, or of the ammonium salt, or of the monoethanolamine salt or of an amino acid salt, of which copolymer is copolymerized either with at least one monomer containing a weak acid function or with at least one neutral monomer, or a copolymer based on 2-methyl-2-[(1-oxo-2-propenyl)amino]-1-propanesulphonic acid partially or totally salified in the form either of an alkali metal salt, or of the ammonium salt, or of the monoethanolamine salt or of an amino acid salt, which copolymer is copolymerized with a monomer containing at least one weak acid function and with at least one neutral monomer, and
(c) the mixture of the said surfactant of water-in-oil (W/O) type and of the said surfactant of oil-in-water (O/W) type has a total HLB number of greater than or equal to 8.5 and less than or equal to 11 and preferably greater than or equal to 9.5 and less than or equal to 10.

2. Composition as defined in Claim 1, in which the monomer comprising a weak acid function is chosen from acrylic acid, methacrylic acid, itaconic acid, maleic acid or 3-methyl-3-[(1-oxo-2-propenyl)amino]butanoic acid, the said acids being partially or totally salified in the form either of an alkali metal salt, or of an ammonium salt, or a monoethanolamine salt or an amino acid salt.

3. Composition as defined in Claim 1, in which the neutral monomer is chosen from acrylamide, 2-hydroxyethyl acrylate, 2,3-dihydroxypropyl acrylate, 2-hydroxyethyl methacrylate and 2,3-dihydroxypropyl methacrylate, or an ethoxylated derivative with a molecular weight of between 400 and 1 000, of each of these esters.

4. Composition as defined in Claim 1, in which the polyelectrolyte is a crosslinked homopolymer of 2-methyl-2-[(1-oxo-2-propenyl)amino]-1-propanesulphonic acid partially or totally salified in the form of the sodium salt or the ammonium salt.

5. Composition as defined in either of Claims 1 and 3, in which the polyelectrolyte is a crosslinked copolymer of 2-methyl-2-[(1-oxo-2-propenyl)amino]-1-propanesulphonic acid partially or totally salified in the form of the sodium salt, the ammonium salt, the monoethanolamine salt or the lysine salt (a) and of 2-hydroxyethyl acrylate (b), in an (a)/(b) molar ratio of between 30/70 and 90/10 and most particularly between 50/50 and 90/10.

6. Composition as defined in either of Claims 1 and 2, in which the polyelectrolyte is a crosslinked copolymer of the sodium salt, the ammonium salt, the monoethanolamine salt or the lysine salt of 2-methyl-2-[(1-oxo-2-propenyl)amino]-1-propanesulphonic acid (a) and of acrylic acid partially or totally salified in the form of the sodium salt, the ammonium salt, the monoethanolamine salt or the lysine salt (c), in an (a)/(c) molar ratio of between 30/70 and 90/10 and most particularly between 30/70 and 45/55.

7. Composition as defined in either of Claims 1 and 3, in which the polyelectrolyte is a crosslinked copolymer of the sodium salt or the ammonium salt of 2-methyl-2-[(1-oxo-2-propenyl)amino]-1-propanesulphonic acid (a) and of acrylamide (d) in an (a)/(d) molar ratio of between 50/50 and 30/70.

8. Composition as defined in Claim 1, in which the polyelectrolyte is a crosslinked copolymer of the sodium salt, the ammonium salt, the monoethanolamine salt or the lysine salt of 2-methyl-2-[(1-oxo-2-propenyl)amino]-1-propanesulphonic- acid (a), of an acrylic acid partially or totally salified in the form of the sodium salt, the ammonium salt, the monoethanolamine salt or the lysine salt (c) and either of acrylamide (d) or 2-hydroxyethyl acrylate (e), in (a)/(c) + (d) or (a)/(c) + (e) molar proportions of between 10/90, 90/10 and (c)/(d) or (c)/(e) molar proportions of between 10/90 and 90/10.

9. Composition as defined in any one of Claims 1 to 8, **characterized in that** the polyelectrolyte is crosslinked and/or branched with a diethylenic or polyethylenic compound in a molar proportion, expressed relative to the monomers used, of from 0.005% to 1%, more particularly from 0.01% to 0.5% and most particularly from 0.1% to 0.25%.

10. Composition as defined in Claim 8, in which the crosslinking agent and/or the branching agent is chosen from diallyloxy acetic acid or a salt thereof, for instance sodium diallyloxyacetate, ethylene glycol dimethacrylate, ethylene glycol diacrylate, diallylurea, trimethylolpropane triacrylate, methylenebis(acrylamide), triallylamine and diallyl tartramide, or a mixture of these compounds.

11. Composition as defined in any one of Claims 1 to 10, in which the oil phase represents from 25% to 50% and more particularly from 30% to 40% of its total weight.

12. Process for preparing a composition as defined in any one of Claims 1 to 11, **characterized in that**:
a) an oil phase and surfactants are added, with stirring to an aqueous solution containing the monomers and the optional additives, so as to form a reverse microemulsion, and then
b) the polymerization reaction is initiated and the said reaction is then left to proceed to form the reverse microlatex.

13. Cosmetic, dermopharmaceutical or pharmaceutical composition, **characterized in that** it comprises at least one composition as defined in any one of Claims 1 to 11.

14. Use of a composition as defined in any one of Claims 1 to 11, as a thickener and/or emulsifier in preparations intended for all kinds of industries, whether it is, for example, the textile industry, the paper industry or the paint manufacturing industry, and more particularly as a thickener for printing pastes based on pigments or dyes.

## Patentansprüche

1. Zusammensetzung, enthaltend eine Ölphase, eine wäßrige Phase, mindestens ein Tensid vom Wasser-in-Öl-Typ (W/O-Typ), mindestens ein Tensid vom Öl-in-Wasser-Typ (O/W-Typ) und einen verzweigten oder vernetzten Polyelektrolyt, **dadurch gekennzeichnet, daß**:
(a) es sich bei der Zusammensetzung um einen selbstinvertierenden inversen Mikrolatex, der 15 bis 40 Gew.-% und vorzugsweise 20 bis 30 Gew.-% des Polyelektrolyts und zwischen 8 und 20 Ges.-% Tenside enthält, handelt,
(b) es sich bei dem Polyelektrolyt entweder um ein Homopolymer von 2-Methyl-2-[(1-oxo-2-propenyl)-amino]-1-propansulfonsäure, teilweise oder vollständig versalzt in Form entweder eines Alkalimetallsalzes oder des Ammoniumsalzes oder des Monoethanolaminsalzes oder eines Aminosäuresalzes, oder ein Copolymer auf Basis von 2-Methyl-2-[(1-oxo-2-propenyl)amino]-1-propansulfonsäure, teilweise oder vollständig versalzt in Form entweder eines Alkalimetallsalzes oder des Ammoniumsalzes oder des Monoethanolaminsalzes oder eines Aminosäuresalzes, copolymerisiert mit mindestens einem Monomer mit einer schwachen Säurefunktion oder mit mindestens einem neutralen Monomer, oder ein Copolymer auf Basis von 2-Methyl-2-[(1-oxo-2-propenyl)amino]-1-propansulfonsäure, teilweise oder vollständig versalzt in Form entweder eines Alkalimetallsalzes oder des Ammoniumsalzes oder des Monoethanolaminsalzes oder eines Aminosäuresalzes, copolymerisiert mit einem Monomer mit mindestens einer schwachen Säurefunktion und mit mindestens einem neutralen Monomer, handelt und
(c) die Mischung des Tensids vom Wasser-in-Öl-Typ (W/O-Typ) und des Tensids vom Öl-in-Wasser-Typ (O/W-Typ) einen Gesamt-HLB-Wert größer gleich 8,5 und kleiner gleich 11 und vorzugsweise größer gleich 9,5 und kleiner gleich 10 aufweist.

2. Zusammensetzung nach Anspruch 1, worin das Monomer mit einer schwachen Säurefunktion unter Acrylsäure, Methacrylsäure, Itaconsäure, Maleinsäure oder 3-Methyl-3-[(1-oxo-2-propenyl)amino]butansäure ausgewählt ist, wobei die Säuren teilweise oder vollständig in Form entweder eines Alkalimetallsalzes oder des Ammoniumsalzes oder des Monoethanolaminsalzes oder eines Aminosäuresalzes versalzt sind.

3. Zusammensetzung nach Anspruch 1, worin das neutrale Monomer unter Acrylamid, 2-Hydroxyethylacrylat, 2,3-Dihydroxypropylacrylat, 2-Hydroxyethylmethacrylat und 2,3-Dihydroxypropylmethacrylat oder einem ethoxylierten Derivat mit einem Molekulargewicht zwischen 400 und 1000 jedes dieser Ester ausgewählt ist.

4. Zusammensetzung nach Anspruch 1, worin es sich bei dem Polyelektrolyt um ein vernetztes Homopolymer von 2-Methyl-2-[(1-oxo-2-propenyl)amino]-1-propansulfonsäure, teilweise oder vollständig versalzt in Form des Natriumsalzes oder Ammoniumsalzes, handelt.

5. Zusammensetzung nach einem der Ansprüche 1 bis 3, worin es sich bei dem Polyelektrolyt um ein vernetztes Copolymer von 2-Methyl-2-[(1-oxo-2-propenyl)amino]-1-propansulfonsäure, teilweise oder vollständig versalzt in Form des Natriumsalzes, des Ammoniumsalzes, des Monoethanolaminsalzes oder des Lysinsalzes, (a) und (2-Hydroxyethyl)acrylat (b) in einem Molverhältnis (a) / (b) zwischen 30 / 70 und 90 / 10 und ganz speziell 50 / 50 und 90 / 10 handelt.

6. Zusammensetzung nach einem der Ansprüche 1 oder 2, worin es sich bei dem Polyelektrolyt um ein vernetztes Copolymer des Natriumsalzes, des Ammoniumsalzes, des Monoethanolaminsalzes oder des Lysinsalzes von 2-Methyl-2-[(1-oxo-2-propenyl)-amino]-1-propansulfonsäure (a) und Acrylsäure, teilweise oder vollständig versalzt in Form des Natriumsalzes, des Ammoniumsalzes, des Monoethanolaminsalzes oder des Lysinsalzes, (c) in einem Molverhältnis (a) / (c) zwischen 30 / 70 und 90 / 10 und ganz speziell 30 / 70 und 45 / 55 handelt.

7. Zusammensetzung nach einem der Ansprüche 1 oder 3, worin es sich bei dem Polyelektrolyt um ein vernetztes Copolymer des Natriumsalzes, 2 oder des Ammoniumsalzes von 2-Methyl-2-[(1-oxo-2-propenyl)-amino]-1-propansulfonsäure (a) und Acrylamid (d) in einem Molverhältnis (a) / (d) zwischen 50 / 50 und 30 / 70 handelt.

8. Zusammensetzung nach Anspruch 1, worin es sich bei dem Polyelektrolyt um ein vernetztes Copolymer des Natriumsalzes, des Ammoniumsalzes, des Monoethanolaminsalzes oder des Lysinsalzes von 2-Methyl-2-[(1-oxo-2-propenyl)amino]-1-propansulfonsäure (a), Acrylsäure, teilweise oder vollständig versalzt in Form des Natriumsalzes, des Ammoniumsalzes, des Monoethanolaminsalzes oder des Lysinsalzes, (c) und entweder Acrylamid (d) oder (2-Hydroxyethyl)acrylat (e) in molaren Anteilen (a) / (c) + (d) oder (a) / (c) + (e) zwischen 10 / 90, 90 / 10 und (c) / (d) oder (c) / (e) zwischen 10 / 90 und 90 / 10 handelt.

9. Zusammensetzung nach einem der Ansprüche 1 bis 8, **dadurch gekennzeichnet, daß** der Polyelektrolyt mit einer diethylenischen oder polyethylenischen Verbindung in einem molaren Anteil, bezogen auf die eingesetzten Monomere, von 0,005 bis 1%, spezieller von 0,01 bis 0,5% und ganz speziell 0,1 bis 0,25% vernetzt und/oder verzweigt ist.

10. Zusammensetzung nach Anspruch 8, worin das Vernetzungs- und/oder Verzweigungsmittel unter Diallyloxyessigsäure oder einem Salz davon, wie Natriumdiallyloxyacetat, Ethylenglykoldimethacrylat, Ethylenglykoldiacrylat, Diallylharnstoff, Trimethylolpropantriacrylat., Methylenbis(acrylamid), Triallylamin, Diallyltartramid oder einer Mischung dieser Verbindungen ausgewählt ist.

11. Zusammensetzung nach einem der Ansprüche 1 bis 10, worin die Ölphase 25 bis 50% und spezieller 30 bis 40% des Gesamtgewichts ausmacht.

12. Verfahren zur Herstellung einer Zusammensetzung nach einem der Ansprüche 1 bis 11, **dadurch gekennzeichnet, daß** man:
a) eine die Monomere und die fakultativen Additive enthaltende wäßrige Lösung unter Rühren mit einer Ölphase und Tensiden versetzt, wobei man eine inverse Mikroemulsion erhält, und dann
b) die Polymerisationsreaktion initiiert und dann die Reaktion ablaufen läßt, wobei man den inversen Mikrolatex erhält.

13. Kosmetische, dermopharmazeutische oder pharmazeutische Zusammensetzung, **dadurch gekennzeichnet, daß** sie mindestens eine Zusammensetzung nach einem der Ansprüche 1 bis 11 enthält.

14. Verwendung einer Zusammensetzung nach einem der Ansprüche 1 bis 11 als Verdickungsmittel und/oder Emulgator in Präparationen für alle Arten von Industrien, ob es sich beispielsweise um die Textilindustrie, die Papierindustrie oder die Anstrichmittelherstellungsindustrie handelt, und spezieller als Verdickungsmittel für Druckpasten auf Basis von Pigmenten oder Farbstoffen.
